(19) Europäisches Patentamt
European Patent Office
Office européen des brevets

(11) EP 3 517 939 A1

(12) EUROPEAN PATENT APPLICATION

(43) Date of publication:
31.07.2019 Bulletin 2019/31

(51) Int Cl.:
G01N 23/20 (2018.01)          A61B 6/00 (2006.01)
G01N 23/201 (2018.01)         G21K 1/06 (2006.01)
G01N 23/041 (2018.01)

(21) Application number: 18153643.4

(22) Date of filing: 26.01.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(71) Applicant: PAUL SCHERRER INSTITUT
5232 Villigen PSI (CH)

(72) Inventors:
• KAGIAS, Matias
  8002 Zürich (CH)
• STAMPANONI, Marco
  5304 Endingen (CH)
• WANG, Zhentian
  5200 Brugg (CH)

(74) Representative: Fischer, Michael
Siemens AG
Postfach 22 16 34
80506 München (DE)

(54) ARRANGEMENT FOR OMNIDIRECTIONAL SCATTERING IMAGING

(57) X-ray scattering imaging can provide complementary information about the unresolved microstructures of a sample. The scattering signal can be accessed with various methods based on coherent illumination, which span from self-imaging to speckle scanning. The directional sensitivity of the existing methods is limited to a few directions on the imaging plane and it requires the scanning of the optical components, or the rotation of either the sample or the imaging setup, if the full range of possible scattering directions is desired. Recently such an invention has been presented. However, the method requires a very high resolution and is only applicable to imaging setups where this is possible, such as synchrotron facilities. The present invention discloses a new arrangement that allows the simultaneous acquisition of the scattering images in all possible directions in a single shot without the need of high resolution or highly coherence sources. This is achieved by a specialized optical element and means of recording the generated fringe with sufficient spatial resolution.

Fig.1

**Description**

The present invention relates to an arrangement for x-rays, in particular hard x-rays, for obtaining quantitative x-ray images from a sample.

Small angle X-ray scattering(SAXS) can provide information about the microstructure of a sample. Understanding the microstructure of a sample can be vital for various applications since it dictates the samples macroscopic mechanical properties. Conventional small angle scattering methods utilize a highly focused beam to probe only a small area of the sample (in the range of tens of micrometres). Meaning that in order to investigate larger areas long acquisition times are required. Additionally, conventional scattering methods require focusing elements with high mechanical stability, rendering the technique not suitable for clinical or industrial environments. Interferometric methods with gratings can provide scattering information of the sample over large areas however the directional sensitivity is usually limited to only one direction. Recently, a variation of the method allowed a simultaneous scattering sensitivity in all the directions of the imaging plane without the need to rotate the sample or gratings. The method was demonstrated on highly coherent synchrotron sources in combination with micrometre resolution detectors. Albeit, the very promising results the translation to incoherent sources and low resolutions detectors is not trivial.

To cope with these limitations a novel optical element would be necessary. Such an element should not require high coherence, in order to make the translation to non-micro focal sources trivial, and should be capable of producing a modulating fringe which can be resolved without high resolution detectors. It should be noted that such large fringes would require up to tens of meters in order to be achieved with conventional gratings previously reported.

These objectives are achieved according to the present invention by an x-ray imaging arrangement according to claim 1 and preferred embodiments according to the dependent claims 2 to 12.

The invention discloses an arrangement for x-rays, in particular hard x-rays, for obtaining quantitative x-ray images from a sample, comprising:

a) an X-ray source, preferably a standard polychromatic X-ray source;
b) an optical element disposed in the x-ray beam;
c) a position-sensitive detector with spatially modulated detection sensitivity having a number of individual pixels;
d) means for recording the images of the detector;
e) means for evaluating the intensities in a single shot image in order to obtain the characteristics of the sample including absorption, differential phase contrast and directional (small-angle) scattering contrast, preferably for specified regions of pixels; and
f) optionally, an aperture array in front of, or embedded into the X-ray source;
g) wherein the optical element is a periodic structure composed of unit cells wherein the unit cells are placed on a Cartesian or Hexagonal grid, said unit cells are composed of circular structures with certain spacing from each other;
h) each circular structure comprising a diffractive substructure wherein the period of the unit cell is P, the width of each circular structure is S and the spacing between consecutive circular structures is D,
i) the sum of S and D is defined as the period of the circular structure within the unit cell and is noted as p=S+D; and wherein
j) the diffractive structures within each circular structure generate a considerable diffraction of the incoming X-rays in order to minimize the transmitted x-ray intensity through the circular structure; preferably achieved by inducing a periodic phase shift or absorption of the incoming x-rays wherein the period of this substructure is noted as $g_1$.

This arrangement does not require high coherence of the x-ray source in order to make the translation to non-micro focal sources trivial and is capable of producing a modulating fringe which can be resolved without high resolution detectors. The term "circular structure" has the meaning of a closed loop structure that can be for example annular, hexagonal or other polygonal or multiangular structure.

Preferably, the circular structure is a concentric annuli or concentric polygons defining diffractive and non-diffractive areas.

Further preferably, the optical element is made by deep etching into silicon, a polymer or similar material, or deposition of a metal into gaps of a low-absorbing structure or growing of metal on low-absorbing substrate, or growing metal or polymer by 3D printing. In either case the height of the metal is optimized for maximizing diffraction efficiency for the given X-ray energy or polychromatic X-ray spectrum.

A further preferred embodiment of the present invention is achieved, when the optical element creates a periodic intensity pattern with a repetition of each unit cell P' and the period within each unit cell is p' at a known distance downstream on the detector; P' and p' match the radius of curvature of an incident wave front by relation $p' = p \frac{d_1 + l_1}{l_1}$,

$P' = P \frac{d_1 + l_1}{l_1}$ where $l_1$ is the distance between the X-ray source (or the aperture array if used) to the optical element, $d_1$ is the distance between the optical element and the x-ray detector.

For the aperture array an advantageous design can be achieved when the aperture array is preferably a 2D repetition of holes with pitch of $P_0 = P' \times \frac{l_1}{d_1}$ or integer multiples thereof, or when the aperture array is not used but the X-ray source comprises 2D array of individual sources that may be mutually incoherent and whose lateral separation $P_0 = P' \times \frac{l_1}{d_1}$ or integer multiples thereof, wherein the width of the apertures in any direction of the imaging plane or the individual source size is less than $p' \times \frac{l_1}{d_1}$ in order not to smear out the intensity pattern completely.

Further, in order to provide a simple arrangement for the sample handling, a mechanism can be comprised to place a sample to be investigated between the X-ray source (or the aperture array if used) and the optical element, or between the optical element and the x-ray detector.

Suitable analysis means may provide for an analysis procedure being implemented for obtaining the absorption, differential phase contrast and directional scattering contrasts of the sample that comprises of the steps of recording two intensity images of the interference pattern (with sample and without sample) on the detector.

Further, the analysis means may comprise means to detect the location of individual unit cells on the recorded flat image by using the circular nature of the optical element, that being said, an intensity maximum is observed in the centre of each unit cell.

Furthermore, the analysis means may comprise means to calculate the shift between the flat and sample images of each unit cell, preferably achieved either with Fourier based methods and/or Hilbert transform methods by calculating the analytical signal or spatial correlation methods.

Further, the analysis means may comprise means to evaluate the radial visibility reduction for every angle in order to obtain omnidirectional scattering images, preferably accomplished by Fourier methods from the following formula

$$C(n, m, \theta) = \frac{R_k^s R_0^f}{R_k^f R_0^f}.$$

Preferably, the mechanism to handle the sample may also comprise means for rotating the sample relatively to the remaining components to perform data collection for a tomographic scan. Additional mechanics can be employed in order to tilt the tomographic axis in the direction of the beam.

Specifically, the detector may preferably comprise resolution enhancement capabilities in terms of introducing virtual pixels smaller than the physical detector pixel size by exploiting the charge sharing effect between neighbouring pixels.

Preferred embodiments of the present invention are hereinafter described in more detail with reference to the attached drawings which depict in:

Figure 1     schematically a drawing of an arrangement for x-ray imaging; (1) X-ray source. In case of a non microfocal X-ray source an aperture array can be used (2). The aperture array can preferably be a Cartesian or hexagonal arrangement of holes. The sample (3) is placed downstream the aperture array. Right after the sample the optical element is placed (4). The x-ray detector (5) is placed at the defined distance from the optical element (4).(6) Zoomed in image of one unit cell, clearly showing the diffractive structure within each annulus;

Figure 2     Directional scattering image of a carbon fibre loop acquired at a synchrotron facility. The colour shows the direction of the underlying structure. The pixel size is 100 x 100 um$^2$;

Figure 3     Directional scattering image of a carbon fiber loop acquired at a compact microfocal X-ray tube. The colour shows the direction of the underlying structure. The pixel size is 200 x 200 um$^2$; and

Figure 4     Scanning electron microscopy image of a single unit cell. Each diffractive annulus has a width of 25 um. The diffractive structure within each diffractive annulus is 1 $\mu$m. The spacing between two consecutive annuli is also 25 $\mu$m.

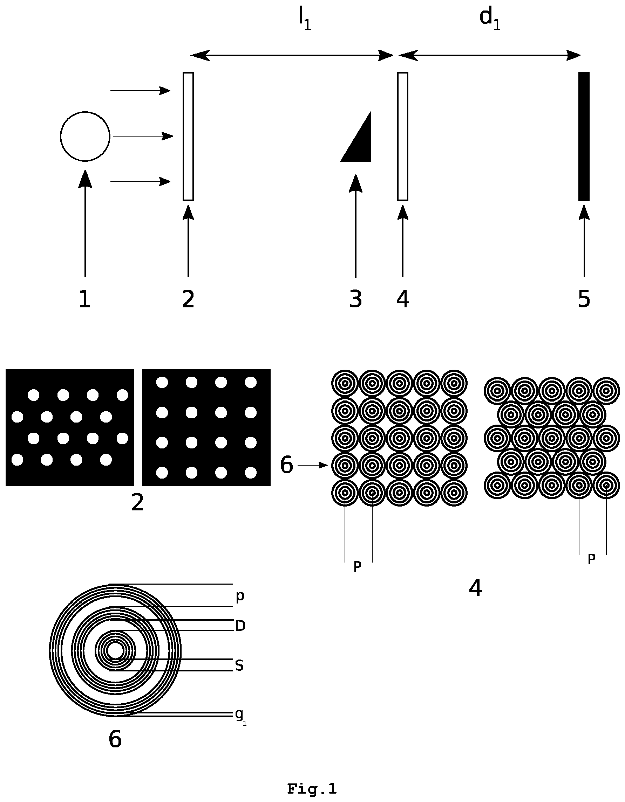

Figure 1 schematically shows an arrangement for single shot x-ray imaging. The arrangement comprises an X-ray source 1, wherein in case of a non-microfocal X-ray source an aperture array 2 can be used. The aperture array 2 can preferably be a Cartesian or hexagonal arrangement of holes. A sample 3 can be placed downstream the aperture array 2. Right after the sample 3, an optical element 4 comprising a number of optically active unit cells is placed. A position sensitive x-ray detector 5 is placed at the defined distance from the optical element 4. With 6 a zoomed-in image of one unit cell is denoted, clearly showing the diffractive structure within each annulus, either on a Cartesian or on a Hexagonal grid.

The single-shot imaging arrangement is capable of omnidirectional scattering sensitivity, acquisition of differential phase contrast signals in vertical and horizontal directions and absorption contrast without the rotation or shift of optical elements or the sample under examination. There is one key component that enables the omnidirectional scattering sensitivity without the need of high-resolution optics or highly coherent x-ray sources: a dedicated unit cell design allowing fringes of larger periods to be generated compared to conventional interferometric methods. The method relies on incoherent superposition of diffracted beams and therefore is suitable for extended and/or polychromatic X-ray sources, like X-ray tubes.

The imaging arrangement comprises the following elements:

- The X-Ray source 1 providing radiation for examining the object of interest.
- The optional aperture array 2 for decreasing the size of the X-Ray source 1 and effectively not smearing out the induced intensity modulation from the optical element 4. The aperture array 2 is manufactured from an absorbing material and preferably is a 2D repetition of holes in order to decrease the projected source size of the source in all directions defined on the imaging plane.
- The optical element 4 that introduces sufficient diffraction of the incoming x-rays. Examples for the dedicated design of the optical element are depicted in Fig. 1 for the unit cell 6.
- The X-Ray and position sensitive detector 5 that is adapted to detect radiation after passing through the sample 3 and the optical element 4 with a resolution sufficient to record the intensity modulation caused by the optical element 4.

The main innovation of the proposed imaging arrangement is the fundamentally novel optical element 4. Fine structures of the sample 3 cause a beam broadening that smears out the intensity modulation caused by the optical element 4. Since the introduced optical element 4 does not require a coherent superposition of the diffracted beams the coherence requirements of the x-ray source 1 are significantly reduced compared to previous grating based methods. Additionally, the period of the induced intensity modulation can be significantly enlarged compared to conventional grating based methods resulting in relax resolution requirements on the x-ray detector 5.

For a design that causes diffraction of the incoming x-ray under an angle $\vartheta$ and a period p of the annuli the following distance is defined $d_m = \left(m - \frac{1}{2}\right)\frac{p}{\tan\theta}$, where m is a natural number. This is the distance from the optical element 4 where the intensity modulation has a maximum visibility for monochromatic radiation and an infinite source to optical element distance. In the case of non-infinite source to detector distance the distance between the optical element 4 and the x-ray detector 5 where the maximum visibility is observed needs to be scaled accordingly. For a distance $l_1$ between source and O1 the detector should be placed at the distance $d_1$ defined as following $d_1 = \frac{d_m l_1}{l_1 - d_m}$.

The intensity fringe at the selected distance can be characterized from the following periodicities: P' and p', where P' the repetition rate of the images of the unit cells and p' the period of the annuli. These periodicities are connected to the mentioned distances as following $p' = p\frac{d_1 + l_1}{l_1}$, $P' = P\frac{d1 + l_1}{l_1}$. The projected period P' defines the pixel size of the reconstructed images of the sample under investigation.

The imaging procedure requires the acquisition of two images. Initially, an image is recorded with only the optical element 4 (and the aperture array 2 if used) being placed in the x-ray beam. This image will be called the flat image (f). As the next step the sample 3 is introduced into the x-ray beam without shifting or removing the optical element 4 and the aperture array 2 if used and a so-called sample image (s) is recorded.

The analysis procedure starts by locating the generated image of the individual unit cells 6 on the pixel matrix of the flat image. Due to the circular nature of each unit cell a maximum is observed in the centre. This maximum is used as a finding criterion for the centres. Once all the centres have been detected a square area of P'×P' around each centre is selected and will be noted with the spatial coordinate (n, m). The fringe of each unit cell 6 is approximated by

$$I(n.m,\rho,\theta) = A(n,m) + B(n,m,\theta)\cos\left(2\pi\frac{\rho}{p'}\right),$$

where A(n, m) denotes the average intensity in the defined area, B(n, m, $\theta$) the angular depend scattering coefficient

and $\rho$, $\theta$ are the local coordinates at the unit cell (n, m). The transmission image is calculated as the ratio (sample over flat) of the average values of the recorded interference patterns:

$$T(n,m) = \frac{\sum_\theta \sum_\rho I_s(n,m,\rho,\varphi)}{\sum_\theta \sum_\rho I_f(n,m,\rho,\varphi)}$$

The differential phase contrast images in horizontal and vertical directions are calculated by estimating the shift of the individual unit cell self-images. This can be done by a number of methods, for instance, spatial correlation estimation. Here, a method is proposed based on a linear square fit of the local estimated phase difference between the sample and flat fringes with a theoretical model that is valid for a sinusoidal approximation of the fringes. If the sample fringe is shifted by (x0, y0) then the local phase difference for one unit cell is given by

$$\Phi(\rho,\varphi,x_0,y_0) = \frac{2\pi}{p'}\left[\sqrt{\rho^2 - x_0^2 - y_0^2 - 2\rho(x_0 cos\varphi + y_0 sin\varphi)} - \rho\right]$$

The experimental local phase shift is calculated by a Hilbert phase retrieval in the x or y direction. The theoretical model is then fitted to the experimental phase and the values x0 and y0 are estimated.

The directional scattering images are obtained by radial Fourier analysis of the recorded circular fringes. The scattering contrast is calculated by the appropriate Fourier coefficient of the radius of the fringe. The ratio of the Fourier coefficients results in the scatter contrast under that specific angle. Specifically, directional scattering images are given by

$$C(n,m,\varphi) = \frac{R_k^s R_0^f}{R_k^f R_0^f}$$

where $R_k$ is the k-th harmonic of the discrete Fourier transform of the recorded fringe in direction $\varphi$ and k=P'/p'.

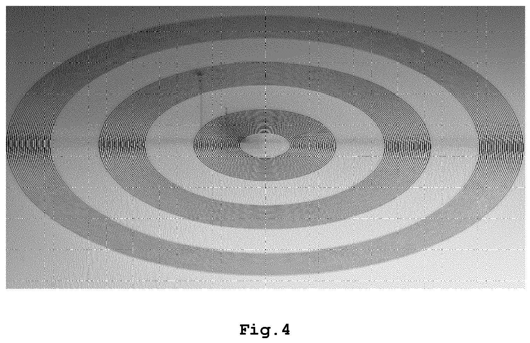

The method was experimentally validated at the TOMCAT beam line of the Swiss Light Source at Paul Scherrer Institut, CH-5232 Villigen PSI, and on a polychromatic X-ray tubes with large and small focal points. The repetition rate of the unit cell of the optical element was 100 $\mu$m, annulus width of 25 $\mu$m, spacing between consecutive annuli was 25 $\mu$m, and the diffractive array of the annuli was achieved by etching trenches of a width of 0.5 $\mu$m with a period of 1 $\mu$m. The trenchers were etched to a depth of 23 $\mu$m which is optimal for a design energy of 17 keV illumination. A scanning electron microscopy (SEM) image of a single unit cell of the optical element can be seen in Fig. 4. The photon energy was selected by a multilayer monochromator. A pco. edge 5.5 CCD camera with a pixel size of 6.5 $\mu$m was placed 34 cm behind the optical element 4.

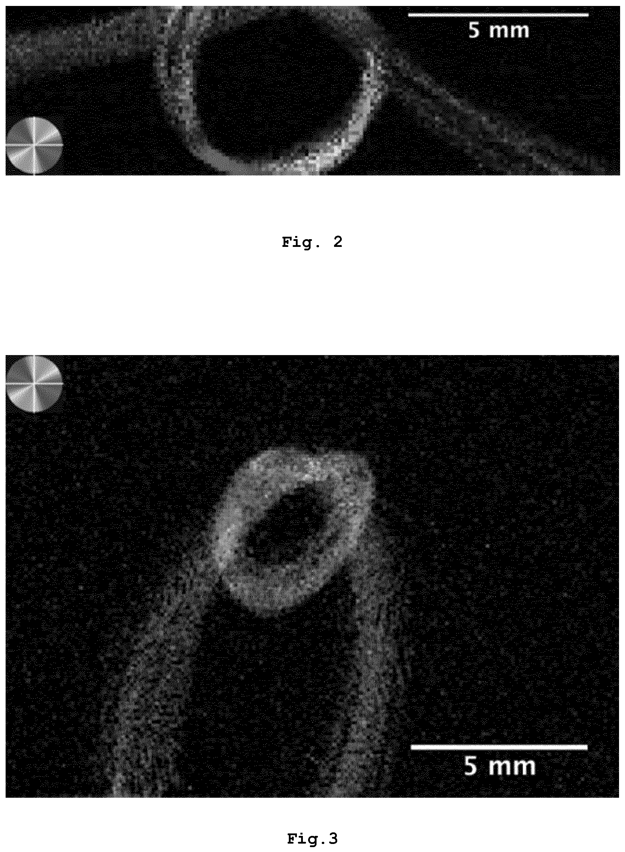

The directional scattering image of the carbon fibre loop imaged on a synchrotron can be seen in Fig. 2. A similar loop shown in Fig. 3 was imaged at a microfocal x-ray tube.

Reference

[1] M. Kagias et al., Phys. Rev. Lett. 116, 093902 (2016)

**Claims**

1. An arrangement for x-rays, in particular hard x-rays, for obtaining quantitative x-ray images from a sample, comprising:

   a) an X-ray source (1), preferably a standard polychromatic X-ray source;
   b) an optical element (4);
   c) a position-sensitive detector (5) with spatially modulated detection sensitivity having a number of individual pixels;
   d) means for recording the images of the detector (5);
   e) means for evaluating the intensities in a single shot image in order to obtain the characteristics of the sample including absorption, differential phase contrast and directional (small-angle) scattering contrast, preferably for specified regions of pixels; and

f) optionally, an aperture array (2) in front of, or embedded into the X-ray source (1);

g) wherein the optical element (4) is a periodic structure composed of unit cells (6) wherein the unit cells (6) are placed on a Cartesian or Hexagonal grid, said unit cells (6) are composed of circular structures with certain spacing from each other;

h) each circular structure comprising a diffractive substructure wherein the period of the unit cell is p, the width of each circular structure is S and the spacing between consecutive circular structures is D,

i) the sum of S and D is defined as the period of the circular structure within the unit cell and is noted as p=S+D; and wherein

j) the diffractive structures within each circular structure generate a considerable diffraction of the incoming X-rays in order to minimize the transmitted x-ray intensity through the circular structure; preferably achieved by inducing a periodic phase shift or absorption of the incoming x-rays wherein the period of this substructure is noted as $g_1$.

2. The arrangement according to claim 1, wherein the circular structure is a concentric annuli or concentric polygons defining diffractive and non-diffractive areas.

3. The arrangement according to claim 1 or 2, wherein the optical element (4) is made by deep etching into silicon, a polymer or similar material, or deposition of a metal into gaps of a low-absorbing structure or growing of metal on low-absorbing substrate, or growing metal or polymer by 3D printing.

4. The arrangement according to any of the preceding claims, wherein the optical element (4) creates a periodic intensity pattern with a repetition of each unit cell P' and the period within each unit cell is p' at a known distance downstream on the detector; P' and p' match the radius of curvature of an incident wave front by relation

$$p' = p\frac{d_1+l_1}{l_1}, \quad P' = P\frac{d_1+l_1}{l_1}$$ where $l_1$ is the distance between the X-ray source (or the aperture array if used) to the optical element, $d_1$ is the distance between the optical element and the x-ray detector.

5. The arrangement according to any of the preceding claims 1 to 4, wherein the aperture array (2) is preferably a 2D repetition of holes with pitch of $P_0 = P' \times \frac{l_1}{d_1}$ or integer multiples thereof, or when the aperture array is not used but the X-ray source comprises 2D array of individual sources that may be mutually incoherent and whose lateral separation $P_0 = P' \times \frac{l_1}{d_1}$ or integer multiples thereof, wherein the width of the apertures in any direction of the imaging plane or the individual source size is less than $p' \times \frac{l_1}{d_1}$.

6. The arrangement according to any of the preceding claims 1 to 5, wherein a mechanism is comprised to place a sample (S) to be investigated between the X-ray source (or the aperture array if used) and the optical element, or between the optical element and the x-ray detector.

7. The arrangement according to any of the preceding claims 1 to 6, wherein an analysis procedure is implemented for obtaining the absorption, differential phase contrast and directional scattering contrasts of the sample that comprises the steps of recording two intensity images of the interference pattern with sample and without sample on the detector (5).

8. The arrangement according to any of the preceding claims, further comprising means to detect the location of individual unit cells (6) on the recorded flat image by using the circular nature of the unit cells (6) of the optical element (4), that being said, an intensity maximum is observed in the centre of a unit cell (6).

9. The arrangement according to any of the preceding claims 1 to 8, further comprising means to calculate the shift between the flat and sample images of each unit cell (6), preferably achieved either with Fourier based methods and/or Hilbert transform methods by calculating the analytical signal or spatial correlation methods.

10. The arrangement according to any of the preceding claims 1 to 9, further comprising means to evaluate the radial visibility reduction for every angle in order to obtain omnidirectional scattering images, preferably accomplished by Fourier methods from the following formula $C(n, m, \theta) = \dfrac{R_k^s R_0^f}{R_k^f R_0^f}$.

11. The arrangement according to any of the preceding claims 1 to 10, comprising means for rotating the sample (3) relatively to the remaining components to perform data collection for a tomographic scan, wherein the orientation of the rotation axis can be arbitrary, preferably adjustable in pitch.

12. The arrangement according to any of the preceding claims 1 to 11, where the detector (5) comprise resolution enhancement capabilities in terms of introducing virtual pixels smaller than the physical detector pixel size by exploiting the charge sharing effect between neighbouring pixels.

$l_1$

$d_1$

1    2    3  4    5

2

6→

4

p
D
S
$g_1$

6

Fig.1

Fig. 2

Fig.3

Fig.4

Europäisches
Patentamt

European
Patent Office

Office européen
des brevets

# EUROPEAN SEARCH REPORT

Application Number

EP 18 15 3643

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/032512 A1 (SCHERRER INST PAUL [CH]) 2 March 2017 (2017-03-02) * page 3, line 1 - page 6, line 10; figures 1-4 * | 1-12 | INV. G01N23/20 A61B6/00 G01N23/201 G21K1/06 G01N23/041 |
| X,D | MATIAS KAGIAS ET AL: "2D-Omnidirectional Hard-X-Ray Scattering Sensitivity in a Single Shot", PHYSICAL REVIEW LETTERS, vol. 116, no. 9, 3 March 2016 (2016-03-03) , XP055495072, US ISSN: 0031-9007, DOI: 10.1103/PhysRevLett.116.093902 * the whole document * | 1-4 | |
| A | WO 2015/168473 A1 (SIGRAY INC [US]) 5 November 2015 (2015-11-05) * paragraph [0099] - paragraph [0108]; figures 10, 11 * | 5 | |
| A | US 2015/071402 A1 (HANDA SOICHIRO [JP]) 12 March 2015 (2015-03-12) * paragraph [0028] - paragraph [0042]; figures 1-3, 5 * | 1-12 | TECHNICAL FIELDS SEARCHED (IPC) G01N A61B G21K |
| A | US 2017/125134 A1 (HANDA SOICHIRO [JP]) 4 May 2017 (2017-05-04) * paragraph [0030] - paragraph [0035]; figures 1A-1C * * paragraph [0049] - paragraph [0055]; figures 5A-5C * | 1-12 | |

The present search report has been drawn up for all claims

| Place of search | Date of completion of the search | Examiner |
|---|---|---|
| The Hague | 26 July 2018 | Pagels, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

# EP 3 517 939 A1

## ANNEX TO THE EUROPEAN SEARCH REPORT
## ON EUROPEAN PATENT APPLICATION NO.

EP 18 15 3643

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2018

| Patent document cited in search report | | Publication date | Patent family member(s) | | Publication date |
|---|---|---|---|---|---|
| WO 2017032512 | A1 | 02-03-2017 | EP<br>EP<br>WO | 3136089 A1<br>3341714 A1<br>2017032512 A1 | 01-03-2017<br>04-07-2018<br>02-03-2017 |
| WO 2015168473 | A1 | 05-11-2015 | CN<br>EP<br>JP<br>WO | 106535769 A<br>3136970 A1<br>2017514583 A<br>2015168473 A1 | 22-03-2017<br>08-03-2017<br>08-06-2017<br>05-11-2015 |
| US 2015071402 | A1 | 12-03-2015 | JP<br>US | 2015072263 A<br>2015071402 A1 | 16-04-2015<br>12-03-2015 |
| US 2017125134 | A1 | 04-05-2017 | JP<br>US | 2017083412 A<br>2017125134 A1 | 18-05-2017<br>04-05-2017 |

EPO FORM P0459

For more details about this annex : see Official Journal of the European Patent Office, No. 12/82

12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. KAGIAS et al.** *Phys. Rev. Lett.,* 2016, vol. 116, 093902